(19) Europäisches Patentamt
European Patent Office
Office européen des brevets

(11) Publication number: **0 041 230**
**A1**

(12) **EUROPEAN PATENT APPLICATION**

(21) Application number: 81104073.2

(22) Date of filing: 27.05.81

(51) Int. Cl.$^3$: C 07 C 43/11
C 08 G 65/28
//C08K5/06

(30) Priority: 30.05.80 US 154851

(43) Date of publication of application: 09.12.81 Bulletin 81/49

(84) Designated Contracting States: BE DE FR GB IT NL SE

(71) Applicant: E.I. DU PONT DE NEMOURS AND COMPANY
Legal Department 1007 Market Street
Wilmington, Delaware 19898(US)

(72) Inventor: Fowell, Peter Alan
202 Painter's Crossing
Chadds Ford Pennsylvania 19317(US)

(74) Representative: Abitz, Walter, Dr.-Ing. et al,
Abitz, Morf, Gritschneder P.O. Box 86 01 09
D-8000 Munich 86(DE)

(54) **Propylene oxide oligomers.**

(57) Propylene oxide oligomers of 2-ethyl hexanol, useful as plasticizers for polyvinyl butyral.

EP 0 041 230 A1

## TITLE

# PROPYLENE OXIDE OLIGOMERS

## BACKGROUND OF THE INVENTION

Polyvinyl butyral is widely used in combination with one or more layers of glass to provide a composite which is resistant to shattering. The polyvinyl butyral typically contains a plasticizer to provide a balance of mechanical properties satisfactory for the subsequent handling and performance requirements. While many different plasticizers have previously been suggested for use in combination with polyvinyl butyral, continual effort has been directed to the discovery of plasticizers which combine outstanding performance characteristics, low cost and applicability to a wide range of glazing materials.

## SUMMARY OF THE INVENTION

The instant invention provides new compositions of matter which are particularly suitable for use as plasticizers in polyvinyl butyral.

Specifically, the instant invention provides propylene oxide oligomers of the general formula:

$$CH_3(CH_2)_3-\overset{CH_2CH_3}{\overset{|}{C}}H-CH_2-O-(CH_2\underset{CH_3}{\underset{|}{C}}H-O)_nH$$

wherein n is an integer of from 1 to 20 and has an average value of about from 3 to 15.

## DETAILED DESCRIPTION OF THE INVENTION

The present invention is based on the discovery of new compositions, the propylene oxide oligomers of 2-ethylhexanol, and their particular suitability as plasticizers for polyvinyl butyral. The compositions have the following general formula

AD-5041

$$\qquad\qquad\qquad CH_2CH_3$$
$$CH_3(CH_2)_3-CH-CH_2-O-(CH_2CH-O)_nH$$
$$\qquad\qquad\qquad\qquad\qquad\qquad\qquad CH_3$$

These compositions can be prepared by first forming the potassium salt of 2-ethylhexanol. The salt can be made by combining the alcohol with potassium metal and appropriate solvent, such as tetrahydrofuran, in an inert atmosphere such as nitrogen. The reaction should be carried on at elevated temperatures, and preferably the reflux temperature of the solvent. The potassium metal is first added to the solvent with vigorous stirring. When the metal is melted and well dispersed, the alcohol is added with additional quantities of solvent. The resulting mixture is then heated at reflux until all of the potassium is reacted, and then cooled to room temperature. The solvent can then be removed under reduced pressure to leave the desired potassium salt in a form of a waxy solid.

The potassium salt of 2-ethylhexanol can be reacted directly with propylene oxide at moderately elevated temperatures of 50-100°C to form the oligomer. The product can be purified by washing with dilute hydrochloric acid, saturated sodium bicarbonate solution and brine. The final product is a colorless to pale yellow liquid at room temperature.

As will be recognized by those skilled in the art, a number of variations in the structure of a particular propylene oxide oligomer of the above formula will be observed. The number of units derived from propylene oxide, indicated by "n", may vary from one molecule to another within an oligomer. In addition, minor quantities of the propylene oxide units, for example, up to about 15%,

can be replaced by ethylene oxide units without changing the basic character of the compound. Moreover, a minor percentage of the methyl groups in the propylene oxide units may be present in the reverse molecular position of that shown in the formula.

The average molecular weight of the propylene oxide oligomers in general is about from 300 to 1000. Oligomers having an average molecular weight below 300 exhibit excessive volatility. Molecular weights in excess of 1000 generally result in incompatibility.

The propylene oxide oligomers are incorporated into polyvinyl butyral as a plasticizer using techniques typical of those applicable to other plasticizers. The polyvinyl butyral and plasticizer are present in quantities which result in a compatible admixture. The quantities of plasticizer which can be added to polyvinyl butyral vary according to the residual hydroxyl content of the polyvinyl butyral as well as the particular oligomer used. In general, less than about 10 pph of plasticizer will not provide any substantial plasticizing effect, while concentrations in excess of 70 pph will result in unsatisfactory structural integrity. Within this range, the maximum concentration of plasticizer will be determined by the concentration at which the particular compound becomes incompatible with the polyvinyl butyral.

As recognized by those skilled in the art, plasticizer incompatibility can result in a haziness in the polyvinyl butyral sheeting, particularly with higher molecular weight plasticizers, and results in diminished tackiness of the film. Incompatibility may also be indicated by plasticizer exuding from the

surface of the finished film. However, in general, a maximum compatible concentration of plasticizer, in parts per hundred parts by weight, can be determined according to the following guidelines. These guidelines apply to oligomers having a narrow molecular weight distribution, that is, wherein the ratio of weight average molecular weight to number average molecular weight is less than about 1.2. The maximum compatible concentrations for oligomers having a broader molecular weight distribution can be readily determined by those skilled in the art.

In general, the maximum compatible concentration can be determined by the formula

$$430 - 10 - \left(\frac{Y}{6}\right)^{1.1}$$

wherein X is the hydroxyl content of the polyvinyl butyral, as percent by weight, calculated as vinyl alcohol, into which the plasticizer is incorporated, and Y is the average molecular weight of the oligomer.

In general, the concentrations indicated by this formula will represent the maximum compatible concentration of plasticizer within 5 pph.

The propylene oxide oligomers of the present invention provide outstanding plasticization of polyvinyl butyral. It has been found that the present compositions are compatible with polyvinyl butyral over a wide range of hydroxyl contents, and provide sheeting with excellent dimensional stability, tensile strength and stiffness. Moreover, the present plasticizers are compatible with other plasticizers used for polyvinyl butyral, including triethyleneglycol di-2-ethylbutyrate (3GH), as well as tetraethyleneglycol di-n-heptanoate (4G7).

The plasticized polyvinyl butyral sheeting can be laminated to one or more layers of glazing

material according to the procedures generally used in the art and illustrated, for example, in U.S. Patents 2,293,656, 3,838,091 and 4,107,366. A particularly surprising advantage of the present invention is that the resulting plasticized polyvinyl butyral sheeting can be used not only with glass, but polymethyl methacrylate and polycarbonate sheeting as well. This is in marked contrast to many polyvinyl butyral plasticizers previously used in the art, which tended to cause undesirable interaction with the plastic materials.

The present invention is further illustrated in the following specific examples, in which parts and percentages are by weight unless otherwise indicated. In the Examples, n in each propylene oxide oligomer molecule is from 1 to 20 and averages about from 3 to 15.

In these Examples, the following tests and procedures were used:

PVB Residual Hydroxyl (Weight Percent as VA): ASTM D1396.

Tensile Strength: ASTM D1708 with samples conditioned and run at 20.6 ± .6°C and 23.5 ± 2% RH instead of 23 ± 2°C and 50 ± 5% RH.

5% Secant Modulus: ASTM D882 with samples conditioned and run at 20.6 ± .6°C and 23.5 ± RH instead of 23 ± 2°C and 50 ± 5% RH.

Pummel Break: SAE J1208

Mean Break Height: ANSI Z26.1 using a staircase method to determine mean break height instead of the one level test of Z26.1 as described in U.S. Patent 3,718,516.

Edge Stability: ANSI Z26.1.

Creep (% Elongation): Test sheeting samples nominally 0.03 inch (0.76 mm) thick are conditioned

for 4 hours at 68 ± 2°F (20.0 ± 1.1°C). The samples are then die cut to 0.75 x 4 inches (1.9 x 10.2 cm), marked and clipped into the test oven at 65 ±°C for 1 hour. A 10 psi (69 kPa) load is applied to each specimen and the elongation measured at 30 minutes. The results are calculated by dividing the gage length after testing by the initial gage length and multiplying by 100.

EXAMPLE 1

Potassium metal (5.58 g, 0.143 mole) was placed in 100 ml of dry tetrahydrofuran under nitrogen. The mixture was heated to the reflux temperature with vigorous stirring. When the potassium had melted and was well dispersed, 2-ethylhexanol (18.5 g, 22.3 ml, 0.143 mole) in 50 ml of dry tetrahydrofuran was added slowly. The mixture was maintained at the reflux temperature until all the potassium had reacted. The mixture was then cooled to room temperature and the solvent was removed under reduced pressure to leave a waxy solid identified as the potassium salt of 2-ethylhexanol.

The solid potassium salt was dissolved in propylene oxide (58 g, 70ml, 1.0 mole) and the solution was transferred under nitrogen into a stainless steel lined pressure vessel. The tube was closed and heated to 70° for 1 hr. followed by 90° for 6 hr. The contents of the vessel were taken up in 250 ml of ether and the ether solution was washed successively with 2-100 ml portions of dilute hydrochloric acid, 2-100 ml portions of saturated sodium bicarbonate, and 2-100 ml portions of brine. The ether layer was dried over magnesium sulfate. The mixture was filtered and the solvent was removed under reduced pressure to yield 69.9g (92%) of a pale yellow liquid. Average molecular weight found

(hydroxyl number) - 400. The product was identified as the propylene oxide oligomer of 2-ethylhexanol, having the formula

$$CH_3(CH_2)_3\ \overset{\overset{CH_2CH_3}{|}}{C}H-CH_2-O-(CH_2\underset{\underset{CH_3}{|}}{C}H-O)_nH$$

wherein n has an average value of about 4.7.

EXAMPLE 2

A propylene oxide was prepared as in EXAMPLE 1, having an average molecular weight of about 425. This oligomer and polyvinyl butyral were added to an 83 mm twin screw Werner and Pfleiderer vented extruder having an L/D ratio of 20:1. The PVB used had a hydroxyl content of about 23%. The ratio of oligomer to PVB was 33/67 (about 49 pph). The extrusion conditions used were a screwspeed 90 rpm, 200°C melt temperature at the exit of the extruder, and 20 in. Hg. vacuum at the vent post, to remove any moisture. 80 ppm potassium formate was added to the melt as an adhesion control agent. A gear pump was used to pump the melt from the extruder to a sheeting die (all at 200°C). Total hold up time was about 14 minutes. A 30-32 mil sheet, 30 inches wide, was extruded from the die into a 20°C water tank to solidify the melt. The sheet was then air dried, powdered with $NaHCO_3$ and wound onto a core.

Laminates were made from this sheet by first washing off the $NaHCO_3$, conditioning at 22% RH/72°F for 8 hours, and laminating to glass washed in water containing 350 ppm $CaCl_2$. Conditions for laminating are 135°C for 9 minutes, at a pressure of 120 psi. Mechanical properties were determined as follows:

| | |
|---|---|
| Tensile strength | 4054 psi |
| Stiffness | 982 psi |
| Pummel break | 7 |
| Mean Break Height (5 lb. ball) | 11.8 ft/0°F; 18.2 ft/72°F; 10.5 ft/100°F |

EXAMPLE 3

The general procedure of Example 2 is repeated, using 48 parts per hundred of the ethyhexyl ended oligomer. Excellent plasticization and compatibility were obtained.

A laminate was prepared from a sheet of the plasticized PVB having a thickness of about from 0.25 to 0.30 mils. The sheeting was laminated between two layers of Lexan polycarbonate by pressing at 140°C and a pressure of 500 psi. The laminate was clear on preparation and remained clear at ambient conditions after 5 days.

EXAMPLE 4

A propylene glycol oligomer of the present invention was put in contact with cast polycarbonate sheeting to test the effect on crazing. In this Example, 1" x 4" strips of Lexan polycarbonate sheeting having a thickness of 1/8" were coated with several drops of the oligomer of EXAMPLE 2.

One hour after coating, each strip was manually flexed through a curve of 80°. A score was then made on the surface of the sheeting and the strip flexed again after 30 minutes. In each case, no crazing was apparent.

COMPARATIVE EXAMPLES A, B, AND C

The general procedure of EXAMPLE 4 was repeated, using, instead of the oligomer of that example, commercially available plasticizers consisting essentially of dihexyl adipate, triethylene glycol di-2-ethyl butyrate, and

tetraethylene glcyol di-n-heptanoate. In each case, the coating with the plasticizer composition resulted in severe haze, crazing and cracking.

I CLAIM:

1. Propylene oxide oligomers of the general formula:

$$\begin{array}{l} \qquad\qquad CH_2CH_3 \\ CH_3(CH_2)_3\cdot\overset{|}{C}H-CH_2-O-(CH_2\underset{|}{C}H-O)_nH \\ \qquad\qquad\qquad\qquad\qquad\quad CH_3 \end{array}$$

wherein n is an integer of from 1 to 20 and has an average value of about from 3 to 15.

European Patent Office

# EUROPEAN SEARCH REPORT

0041230

Application number

EP 81104073.2

| Category | Citation of document with indication, where appropriate, of relevant passages | Relevant to claim | CLASSIFICATION OF THE APPLICATION (Int. Cl.3) |
|---|---|---|---|
| | US - A - 2 520 611 (FREDERICK HOFFMANN ROBERTS)<br>+ Columns 1-3 +<br>-- | 1 | C 07 C 43/11<br>C 08 G 65/28<br>//C 08 K 5/06 |
| | GB - A - 1 371 770 (SANDOZ)<br>+ Example +<br>-- | 1 | |
| | GB - A - 1 384 957 (PECHINEY UGINE KUHLMAN)<br>+ Example 3, lines 27-33 +<br>---- | 1 | |

TECHNICAL FIELDS SEARCHED (Int Cl.3)

C 07 C 43/00
C 08 G 65/00
C 08 K

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 14-08-1981 | HEIN |

EPO Form 1503.1 06.78